# EUROPEAN PATENT SPECIFICATION

(11) **EP 1 150 654 B1**
(45) Date of publication and mention of the grant of the patent: **27.09.2006**
(21) Application number: 00903836.5
(22) Date of filing: 15.02.2000
(51) Int. Cl.: A61Q 19/00

(54) **COSMETIC PRODUCTS COMPRISING CREAM OF TARTAR AND SODIUM BICARBONATE**
WEINSTEIN UND NATRIUMBICARBONAT ENTHALTENDE KOSMETISCHE ZUBEREITUNGEN
PRODUITS COSMETIQUES CONTENANT DE LA CREME DE TARTRE ET DU BICARBONATE DE SODIUM

(30) Priority: 15.02.1999 GB 9903425
(43) Date of publication of application: 07.11.2001
(73) Proprietor: Lush Limited, Dorset BH15 1AB (GB)
(72) Inventor: AMBROSEN, Helen, Dorset BH15 1AB (GB)
(74) Representative: Kenyon, Sarah Elizabeth
(86) International application number: PCT/GB2000/000515
(87) International publication number: WO 2000/047181

(56) References cited:
- FR-A- 1 540 469
- FR-A- 2 549 723
- GB-A- 1 492 660
- GB-A- 1 507 356
- US-A- 3 629 468
- US-A- 4 127 645
- DATABASE WPI Section Ch, Week 197830 Derwent Publications Ltd., London, GB; Class D21, AN 1978-54399A XP002136207 & JP 53 069838 A (LION FAT & OIL CO LTD), 21 June 1978 (1978-06-21)
- PATENT ABSTRACTS OF JAPAN vol. 011, no. 063 (C-406), 26 February 1987 (1987-02-26) & JP 61 225117 A (KAO CORP), 6 October 1986 (1986-10-06)

## Description

The present invention relates to surfactants particularly those for use in contact with the human or animal body.

There is an increasing awareness of environmental issues particularly with regard to the disposal of product packaging and the like. The present invention has been made against this background.

Surfactants are conventionally supplied in containers made, for example, of a rigid or semi-rigid plastics material. The container adds significantly to the cost of the product and environmental pollution is caused by the disposal of empty containers.

US patent 3,629,468 (Andersen) discloses a mouthwash tablet comprising sodium bicarbonate, a surfactant, adipic acid and tartaric acid.

According to one aspect of the present invention there is provided a method of manufacturing a surfactant product for use in contact with the human or animal body and having the form of a solid and comprising cream of tartar, sodium bicarbonate and a surfactant, the method comprising preparing a mixture including 25% to 30% by weight of cream of tartar, 50% to 60% by weight of sodium bicarbonate and 10% to 25% by weight of the liquid surfactant, and allowing the mixture to harden to provide the cosmetic product in the form of a solid.

In addition to the already mentioned beneficial reduction in costs and avoidance of environmental pollution, the present invention can provide appealing novelty items.

It has surprisingly been found that the simple mixture of conventional liquid bubble bath with sodium bicarbonate and cream of tartar produces a relatively stable solid form product. The product is remarkably stable, for example, in not rapidly reacting with water in the atmosphere at normal indoor humidity levels.

Effervescent bath tablets have been known for many years. Such bath tablets can contain sodium bicarbonate and it has also been proposed to provide a bath product comprising a mixture of citric acid and sodium bicarbonate. The product resulting from the mixture of cream of tartar and sodium bicarbonate has, however, very different in characteristics from those of a citric acid and sodium bicarbonate mixture. A mixture containing sodium bicarbonate and citric acid will effervesce strongly when immersed in water and dissolve quickly. That is, they are highly unstable in the presence of water, as is required by their intended use. It is therefor highly surprising that a mixture consisting of sodium bicarbonate and cream of tartar provide a solid material which is highly stable. It is even more surprising that the formation of a stable solid material can be retained when a conventional liquid bubble bath is added to the cream of tartar and sodium bicarbonate mixture.

A mixture of 60 % sodium bicarbonate, 30% cream of tartar and 10% surfactant forms a paste which hardens into a solid over a period of a few hours. The resultant solid material can be broken with the hands and thus small pieces of a solid bar of bubble bath can be broken-off and added to a bath as required. The material is thus in stark contrast to the known effervescent bath tablets which disintegrate if an attempt is made to break-off small pieces in the manner just described.

As mentioned above, in preparation the product is in the form of a paste which subsequently hardens in to a stable solid. The final product can thus very easily be formed in many useful and/or novelty shapes. Moreover, there is no requirement for a rigid plastics container, which reduces costs and avoids environmental pollution caused by the disposal of empty containers.

The relative ratios of the components is for the sodium bicarbonate to constitute 50% to 60% of the initial mixture, cream of tartar 25% to 30% and surfactant 10% to 25%. Small amounts of additives may be included, such as a fragrance and/or colourant.

Although the above description has been of a solid bubble bath as an example of a surfactant, it will be appreciated that the invention is equally well suited to other surfactants/uses. As specific examples mention is made of replacement of the conventional liquid bubble bath by conventional: shampoos, shower gels, toothpaste, facial washes etc. Thus, the surfactant will normally be a foaming agent.

Cream of tartar is readily available and has be used in the above examples for ease of reference. Cream of tartar is of course a form of tartaric acid, itself also known as dihydroxybutanedioic acid, a dicarboxylic acid and one of the most widely distributed of plant acids. Cream of tartar is a common name for potassium hydrogen tartar. Other common names include Crystals of Argolis. The substance is usually obtained from byproducts of wine fermentation. In partially purified form, tartar was known to the ancient Greeks and Romans and the free acid was first isolated in 1769. Its use with a surfactant is, however, not known to have been previously proposed.

Beneficial additives can include one or more essential oils. Colouring additives can also be used to provide not only a general aesthetic appeal but also marketing possibilities by incorporating a brand name or the like with an effect similar to the lettering in edible rock or candy. This can be achieved by moulding the letters from a paste incorporating a colouring additive and then moulding the paste of the main body around the individual letters.

## Claims

1. A method of manufacturing a surfactant product for use in contact with the human or animal body and having the form of a solid and comprising cream of tartar, sodium bicarbonate and a surfactant, the method comprising preparing a mixture including 25% to 30% by weight of cream of tartar, 50% to 60% by weight of sodium bicarbonate and 10% to 25% by weight of the liquid surfactant, and allowing the mixture to harden to provide the cosmetic product in the form of a solid.

2. A method as claimed in claim 1 wherein the mixture includes one or more additives.

3. A method as claimed in claim 2 wherein the mixture includes a fragrance as an additive.

4. A method as claimed in claim 2 or 3 wherein the mixture includes a colouring additive providing lettering or the like in the product.

5. A method as claimed in any one of claims 1 to 4 wherein the liquid surfactant comprises any one of a bubble bath, shampoo, shower gel, toothpaste or facial wash.

## Patentansprüche

1. Verfahren zur Herstellung einer Tensidzubereitung für Verwendung in Kontakt mit dem menschlichen oder tierischen Körper und in Form eines Feststoffes und Weinstein, Natriumbicarbonat und ein Tensid umfassend, wobei das Verfahren ein Zubereiten einer 25 Gew.-% bis 30 Gew.-% Weinstein, 50 Gew.-% bis 60 Gew.-% Natriumbicarbonat und 10 Gew.-% bis 25 Gew.-% eines flüssigen Tensids enthaltenden Mischung und ein Härten lassen der Mischung zur Bereitstellung der kosmetischen Zubereitung in Form eines Feststoffs umfasst.

2. Verfahren nach Anspruch 1, wobei die Mischung einen oder mehrere Zusatzstoffe enthält.

3. Verfahren nach Anspruch 2, wobei die Mischung einen Duftstoff als Zusatzstoff enthält.

4. Verfahren nach Anspruch 2 oder 3, wobei die Mischung einen farbgebenden Zusatzstoff enthält, der eine Beschriftung oder dergleichen im Produkt bereitstellt.

5. Verfahren nach einem der Ansprüche 1 bis 4, wobei das flüssige Tensid beliebig Schaumbad, Shampoo, Duschgel, Zahnpaste oder Gesichtswaschmittel enthält.

## Revendications

1. Procédé de fabrication d'un produit surfactant destiné à être utilisé au contact du corps humain ou animal et étant sous une forme solide et comprenant de la crème de tartre, du bicarbonate de sodium et un surfactant, le procédé comprenant la préparation d'un mélange comprenant de 25 % à 30 % en poids de crème de tartre, de 50 % à 60 % en poids de bicarbonate de sodium et de 10 % à 25 % en poids du surfactant liquide, et le fait de laisser le mélange durcir pour fournir le produit cosmétique sous une forme solide.

2. Procédé selon la revendication 1, dans lequel le mélange comprend un ou plusieurs additifs.

3. Procédé selon la revendication 2, dans lequel le mélange comprend un parfum en tant qu'un additif.

4. Procédé selon la revendication 2 ou 3, dans lequel le mélange comprend un additif colorant formant des lettres ou équivalents dans le produit.

5. Procédé selon l'une quelconque des revendications 1 à 4, dans lequel le surfactant liquide comprend l'un quelconque d'un bain moussant, d'un shampoing, d'un gel douche, d'un dentifrice ou d'un produit nettoyant pour le visage.
